(19) Europäisches Patentamt
European Patent Office
Office européen des brevets

(11) EP 3 714 876 A1

# (12) EUROPÄISCHE PATENTANMELDUNG

(43) Veröffentlichungstag:
**30.09.2020 Patentblatt 2020/40**

(21) Anmeldenummer: **19166138.8**

(22) Anmeldetag: **29.03.2019**

(51) Int Cl.:
***A61K 9/08*** (2006.01) ***A24D 3/14*** (2006.01)
***A24F 47/00*** (2020.01) ***A61K 47/12*** (2006.01)
***A61K 47/20*** (2006.01) ***A61K 47/24*** (2006.01)

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**
Benannte Erstreckungsstaaten:
**BA ME**
Benannte Validierungsstaaten:
**KH MA MD TN**

(71) Anmelder: **VitaSalts AG**
**9496 Balzers (LI)**

(72) Erfinder: **Endler, Stephan**
**8232 Merishausen (CH)**

(74) Vertreter: **Michalski Hüttermann & Partner Patentanwälte mbB**
**Speditionstraße 21**
**40221 Düsseldorf (DE)**

## (54) VERDAMPFUNGSZUSAMMENSETZUNGEN ZUM INHALIEREN

(57) Die vorliegende Erfindung betrifft Verdampfungszusammensetzungen zum Inhalieren, sowie die Verwendung von Verdampfungszusammensetzungen zum Inhalieren. Die Verdampfungszusammensetzungen zum Inhalieren bestehen zumindest aus: Nikotin, zumindest einem Hilfsstoff, optional Aroma, und als Rest einer Basisflüssigkeit, wobei der zumindest eine Hilfsstoff einen ersten pKs-Wert aufweist in einem Bereich von größer oder gleich -5 bis kleiner oder gleich 10, vorzugsweise größer oder gleich -2 bis kleiner oder gleich 5, besonders bevorzugt größer oder gleich -2 bis kleiner oder gleich 4.

EP 3 714 876 A1

## Beschreibung

Die vorliegende Erfindung betrifft Verdampfungszusammensetzungen zum Inhalieren, sowie die Verwendung von Verdampfungszusammensetzungen zum Inhalieren.

Verdampfungszusammensetzungen zum Inhalieren sind an sich bekannt und finden insbesondere im Bereich elektronischer Zigaretten Anwendung. Dabei werden Flüssigkeiten mit elektronischen Zigaretten verdampft. Der Dampf wird von einem Konsumenten üblicherweise durch den Mund in die Lungen inhaliert. Von besonderem Interesse sind dabei nikotinhaltige Verdampfungszusammensetzungen, wobei das Nikotin durch Inhalation des Dampfes in den Blutkreislauf gelangt um seine Wirkung zu entfalten.

Verdampfungszusammensetzungen zum Inhalieren können noch Verbesserungspotential bieten. Verbesserungspotential kann sich dabei insbesondere in der Resorbierbarkeit und Resorptionsgeschwindigkeit des Nikotins, in der Verdampfbarkeit des Nikotins und in der Lagerstabilität der Verdampfungszusammensetzungen zum Inhalieren ergeben.

Es ist daher die Aufgabe der vorliegenden Erfindung, eine verbesserte Verdampfungszusammensetzung zum Inhalieren bereitzustellen.

Gelöst wird diese Aufgabe durch Verdampfungszusammensetzungen zum Inhalieren gemäß Anspruch 1 sowie ferner durch die Verwendung einer Verdampfungszusammensetzungen zum Inhalieren gemäß Anspruch 15. Bevorzugte Ausgestaltungen der Erfindung sind in den Unteransprüchen, in der Beschreibung oder in den Beispielen angegeben, wobei weitere in den Unteransprüchen oder in der Beschreibung oder den Beispielen beschriebene oder gezeigte Merkmale einzeln oder in einer beliebigen Kombination einen Gegenstand der Erfindung darstellen können, wenn sich aus dem Kontext nicht eindeutig das Gegenteil ergibt.

Mit der Erfindung wird eine Verdampfungszusammensetzung zum Inhalieren vorgeschlagen.

Die Verdampfungszusammensetzung zum Inhalieren besteht zumindest aus

- Nikotin,
- zumindest einem Hilfsstoff,
- optional Aroma,
- und als Rest einer Basisflüssigkeit,

wobei der zumindest eine Hilfsstoff einen ersten pKs-Wert aufweist in einem Bereich von größer oder gleich -5 bis kleiner oder gleich 10, vorzugsweise größer oder gleich -2 bis kleiner oder gleich 5, besonders bevorzugt größer oder gleich -2 bis kleiner oder gleich 4.

Es konnte in überraschender Weise gezeigt werden, dass die erfindungsgemäßen Verdampfungszusammensetzungen zum Inhalieren eine verbesserte Resorbierbarkeit und Resorptionsgeschwindigkeit des Nikotins aufweisen. Zudem konnte in überraschender Weise gezeigt werden, dass die Verdampfungszusammensetzungen zum Inhalieren eine verbesserte Verdampfbarkeit des Nikotins und eine verbesserte Lagerstabilität aufweisen. Zudem kann in überraschender Weise der Geschmackseinfluss des Nikotins und der Throat-Hit beim Inhalieren eingestellt werden.

Unter Nikotin ist im Rahmen der vorliegenden Erfindung das natürlich in den Blättern der Tabakpflanze vorkommende Alkaloid zu verstehen, das auch unter den Namen (S)-(-)-3-(1-Methyl-pyrrolidin-2-yl)pyridin, (S)-(-)-1-Methyl-2-(3-pyridyl)pyrrolidin, oder L-3-Pyridyl-N-methylpyrrolidin bekannt ist.

Unter Aroma ist im Sinne der vorliegenden Erfindung eine Verbindung oder eine Mischung von Verbindungen zu verstehen, die einen vorzugsweise angenehmen, charakteristischen Geschmack oder Geruch aufweist.

Unter Basisflüssigkeit ist im Sinne der vorliegenden Erfindung eine Flüssigkeit zu verstehen, die verdampfbar ist und in der das Nikotin, der zumindest eine Hilfsstoff und optional das Aroma im Wesentlichen lösbar sind.

Unter einem pKs-Wert ist im Sinne der vorliegenden Erfindung der negative dekadische Logarithmus der Säurekonstanten Ks zu verstehen, die die Lage des Gleichgewichts der Reaktion des Hilfsstoffes mit Wasser unter Protolyse angibt. Für einen Hilfsstoff HA und die Gleichgewichtsreaktion von HA durch Protolyse mit Wasser zu $A^-$ und $H_3O^+$ ist der pKs-Wert definiert als:

$$pKs = -\lg\left(\frac{c(H_3O^+) * c(A^-)}{c(HA)} * 1\frac{L}{mol}\right)$$

wobei $c(H_3O^+)$ die Konzentration von $H_3O^+$, $c(A^-)$ die Konzentration von deprotoniertem Hilfsstoff, und c(HA) die Konzentration von nicht deprotoniertem Hilfsstoff im Gleichgewicht in mol/L ist.

Unter Resorbierbarkeit ist im Sinne der vorliegenden Erfindung insbesondere die Menge resorbierten Nikotins im Verhältnis zur Menge inhalierten Nikotins zu verstehen.

Unter Throat-Hit ist im Sinne der vorliegenden Erfindung das kratzende Gefühl im Rachen beim Inhalieren von Dampf der Verdampfungszusammensetzungen zu verstehen.

Ohne an eine Theorie gebunden zu sein wird davon ausgegangen, dass durch die erfindungsgemäßen Hilfsstoffe eine mögliche Lösungsmittelhülle von Molekülen der Basisflüssigkeit um das Nikotin aufgebrochen werden kann, da sich die Polarität des Nikotins durch die Protonierung stark verändert und insbesondere auch ein Stickstoffatom des Nikotins als Wasserstoffbrückenakzeptor blockiert wird. Zudem wird ohne an eine Theorie gebunden zu sein davon ausgegangen, dass zumindest eine Anzahl an Nikotinmolekülen bei der Resorption beispielsweise durch intermolekulare Wechselwirkungen wie Wasserstoffbrückenbindungen jeweils an Moleküle des deprotonierten Hilfsstoffes gebunden ist und gemeinsam mit dem Hilfsstoff resorbiert wird. Dadurch kann beispielsweise je nach Präferenz des Konsumenten der Throat-Hit verringern oder verstärkt werden oder die Resorptionsgeschwindigkeit des Nikotins erhöhen oder Verringert werden, um entweder ein stärkeres Rauschgefühl oder eine kontinuierlichere Beruhigung zu erreichen.

Eine vorbeschrieben Verdampfungszusammensetzungen zum Inhalieren dient somit insbesondere zur Verbesserung der Resorbierbarkeit und Resorptionsgeschwindigkeit des Nikotins beim Inhalieren eines Dampfes der Verdampfungszusammensetzung und einer verbesserten Verdampfbarkeit des Nikotins, sowie einer verbesserten Lagerstabilität der Verdampfungszusammensetzung. Zudem dient die vorbeschriebenen Verdampfungszusammensetzung zum Inhalieren dem Einstellen des Geschmackseinflusses des Nikotins und des Throat-Hits beim Inhalieren.

Die Verdampfungszusammensetzung zum Inhalieren kann durch Zusammengeben der einzelnen Bestandteile unter Rühren und optional unter Erwärmung mit üblichen Verfahren hergestellt werden.

Die Verdampfungszusammensetzung zum Inhalieren kann beispielsweise mit einer üblichen elektronischen Zigarette verdampft werden und der entstandene Dampf kann vom Konsumenten auf bekannte Weise inhaliert werden.

In einer bevorzugten Ausgestaltung der Erfindung kann vorgesehen sein, dass der zumindest eine Hilfsstoff einen zweiten und optional dritten pKs-Wert aufweist in einem Bereich von größer oder gleich 0 bis kleiner oder gleich 10, vorzugsweise größer oder gleich 2 bis kleiner oder gleich 8, besonders bevorzugt größer oder gleich 3,5 bis kleiner oder gleich 6,5.

Dadurch kann vorteilhafter Weise erreicht werden, dass weniger Hilfsstoff verwendet werden muss. Zudem kann dadurch insbesondere die Resorbierbarkeit des Nikotins weiter eingestellt werden.

Unter einem zweiten pKs-Wert ist im Sinne der vorliegenden Erfindung der negative dekadische Logarithmus der zweiten Säurekonstanten Ks zu verstehen, die die Lage des Gleichgewichts der weiteren Reaktion des bereits deprotonierten Hilfsstoffes mit Wasser unter Protolyse angibt. Unter einem dritten pKs-Wert ist im Sinne der vorliegenden Erfindung entsprechend der negative dekadische Logarithmus der dritten Säurekonstanten Ks zu verstehen, die die Lage des Gleichgewichts der weiteren Reaktion des bereits zweifach deprotonierten Hilfsstoffes mit Wasser unter Protolyse angibt.

In einer bevorzugten Ausgestaltung der Erfindung kann vorgesehen sein, dass die Zusammensetzung das Nikotin in einer Menge in einem Bereich von größer oder gleich 1 mg/mL bis kleiner oder gleich 100 mg/mL bezogen auf die Zusammensetzung aufweist, vorzugsweise in einem Bereich von größer oder gleich 2 mg/mL bis kleiner oder gleich 20 mg/mL, beispielsweise in einem Bereich von größer oder gleich 4 mg/mL bis kleiner oder gleich 10 mg/mL.

Dadurch kann vorteilhafter Weise eine Nikotinkonzentration erreicht werden, durch die beim Konsum das Nikotin eine ausreichende physiologische Wirkung erzielt, ohne Rachen und Lungen übermäßig zu reizen.

In einer bevorzugten Ausgestaltung der Erfindung kann vorgesehen sein, dass die Zusammensetzung ein molares Verhältnis von Hilfsstoff zu Nikotin aufweist in einem Bereich von größer oder gleich 0,25:n bis kleiner oder gleich 4:n, vorzugsweise in einem Bereich von größer oder gleich 0,5:n bis kleiner oder gleich 2:n, besonders bevorzugt in einem Bereich von größer oder gleich 0,9:n bis kleiner oder gleich 1,1:n, wobei n die Anzahl der pKs-Werte des zumindest einen Hilfsstoffes nach einem der vorherigen Ansprüche ist.

Dadurch kann vorteilhafter Weise der Anteil protonierten Nikotins bezogen auf die Stoffmenge des Nikotins in der Verdampfungszusammensetzung zum Inhalieren eingestellt werden. Zudem kann dadurch vorteilhafter Weise eingestellt werden, ob die Zusammensetzung basisch, sauer oder neutral reagiert. Beispielsweise kann die Zusammensetzung schwach sauer, schwach basisch oder neutral reagieren.

In einer bevorzugten Ausgestaltung der Erfindung kann vorgesehen sein, dass die Zusammensetzung den zumindest einen Hilfsstoff in einer Menge aufweist, durch die größer oder gleich 50% des Nikotins protoniert vorliegen, vorzugsweise größer oder gleich 70%, besonders bevorzugt größer oder gleich 90%, bezogen auf die Stoffmenge des Nikotins.

Dadurch kann vorteilhafter Weise eine besonders veränderte Resorbierbarkeit des Nikotins erreicht werden.

In einer bevorzugten Ausgestaltung der Erfindung kann vorgesehen sein, dass die Zusammensetzung ein molares Verhältnis von Hilfsstoff zu Nikotin aufweist in einem Bereich von größer oder gleich 2:n bis kleiner oder gleich 4:n, wobei n die Anzahl der pKs-Werte des zumindest einen Hilfsstoffes ist.

Dadurch kann vorteilhafter Weise erreicht werden, dass besonders große Anteile der Nikotinmoleküle protoniert sind. Insbesondere kann dadurch auch erreicht werden, dass zumindest einige der Nikotinmoleküle zweifach protoniert sind.

In einer bevorzugten Ausgestaltung der Erfindung kann vorgesehen sein, dass der zumindest eine Hilfsstoff einen Siedepunkt und/oder Zersetzungspunkt in einem Bereich aufweist von größer oder gleich 80°C bis kleiner oder gleich 450°C, vorzugsweise größer oder gleich 90°C bis kleiner oder gleich 400°C, besonders bevorzugt von größer oder gleich 100°C bis kleiner oder gleich 300°C, insbesondere von größer oder gleich 110°C bis kleiner oder gleich 160°C.

Dadurch kann vorteilhafter Weise erreicht werden, dass sich der Hilfsstoff auch besonders gut mit der Verdampfungszusammensetzung verdampfen lässt. Insbesondere kann dadurch erreicht werden, dass die

Konzentration des Hilfsstoffes im Dampf der Verdampfungszusammensetzung sich nur unwesentlich von der Konzentration des Hilfsstoffes in der unverdampften Verdampfungszusammensetzung unterscheidet. Beispielsweise kann sich die Konzentration um weniger als 10%, bevorzugt weniger als 5%, insbesondere weniger als 1% unterscheiden.

In einer bevorzugten Ausgestaltung der Erfindung kann vorgesehen sein, dass der zumindest ein Hilfsstoff eine molare Masse aufweist in einem Bereich von größer oder gleich 40 g/mol bis kleiner oder gleich 320 g/mol, vorzugsweise in einem Bereich von größer oder gleich 60 g/mol bis kleiner oder gleich 150 g/mol.

Dadurch kann vorteilhafter Weise erreicht werden, dass der Hilfsstoff die Resorption besonders gut unterstützt.

In einer bevorzugten Ausgestaltung der Erfindung kann vorgesehen sein, dass der Taupunkt der Verdampfungszusammensetzung weniger als 10°C größer als der Siedepunkt der Verdampfungszusammensetzung ist, vorzugsweise weniger als 5°C größer, insbesondere weniger als 1°C größer.

Dadurch kann vorteilhafter Weise erreicht werden, dass die Konzentration des Hilfsstoffes und insbesondere des Nikotins im Dampf der Verdampfungszusammensetzung sich nur unwesentlich von der Konzentration des Hilfsstoffes in der unverdampften Verdampfungszusammensetzung unterscheidet. Beispielsweise kann sich die Konzentration um weniger als 10%, bevorzugt weniger als 5%, insbesondere weniger als 1% unterscheiden.

In einer bevorzugten Ausgestaltung der Erfindung kann vorgesehen sein, dass der zumindest eine Hilfsstoff ausgewählt ist aus der Gruppe bestehend aus Carbonsäuren, Sulfonsäuren, Schwefelsäureestern, Phosphonsäuren, Phosphorsäureestern, CH-aciden Verbindungen, NH-aciden Verbindungen, Enolen, Phenolen, oder Mischungen davon.

Es konnte in überraschender Weise gezeigt werden, dass sich diese Stoffe besonders gut als Hilfsstoffe eignen.

In einer bevorzugten Ausgestaltung der Erfindung kann vorgesehen sein, dass der zumindest eine Hilfsstoff eine Carbonsäure der folgenden Formel ist:

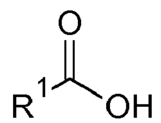

wobei $R^1$ H, -OH, -COOH, C1-8-Alkyl, C2-8-Alkenyl, C1-8-Ketoalkyl, C1-8-Aminoalkyl, Pyridyl, Furan, C1-8-Carboxyalkyl, C2-8-Carboxyalkenyl, C1-8-Ketocarboxyalkyl, C1-8-Aminocarboxalkyl, C1-8-Hydroxycarboxyalkyl, C1-8-Dicarboxyalkyl, C1-8-Hydroxydicarboxyalkyl, N-Neuraminsäure oder zyklisches Hydroxymethyltriterpenoidyl ist.

Es konnte in überraschender Weise gezeigt werden, dass sich diese Stoffe besonders gut als Hilfsstoffe eignen. Insbesondere weisen diese Stoffe in vorteilhafter Weise eine gut abschätzbare Reaktivität auf. Dadurch kann eine Lagerstabilität gut eingeschätzt werden.

In einer bevorzugten Ausgestaltung der Erfindung kann vorgesehen sein, dass $R^1$ C2-5-Alkyl, C2-3-Alkenyl oder C1-3-alpha-Ketoalkyl ist.

In einer bevorzugten Ausgestaltung der Erfindung kann vorgesehen sein, dass $R^1$ C3-4-Carboxyalkyl, C2-3-Carboxyalkenyl, C1-3-alpha-Ketocarboxyalkyl, C2-3-Aminocarboxalkyl, C3-5-Hydroxycarboxyalkyl, C3-5-Dicarboxyalkyl oder C3-5-Hydroxydicarboxyalky ist.

In einer bevorzugten Ausgestaltung der Erfindung kann vorgesehen sein, dass der zumindest eine Hilfsstoff eine Carbonsäure ist, ausgewählt aus der Gruppe bestehend aus Glyoxylsäure, Propansäure, Maleinsäure, 3-Butensäure, 2-Oxobutansäure, Methylmalonsäure, Itaconsäure, 2-Oxoglutarsäure, Dimethylmalonsäure, Glutarsäure, Methylbernsteinsäure, Adipinsäure, D-Glucarsäure, Hexansäure, Liponsäure, Furan-2-carbonsäure, Acrylsäure, Picolinsäure, Isocitronensäure, 4-Methylvaleriansäure, Asparaginsäure, Glutaminsäure, N-Acetylneuraminsäure, Niacin, Benztraubensäure, Milchsäure, Fumarsäure, Oxalessigsäure, Bernsteinsäure, Buttersäure, Lävulinsäure, Citronensäure, Gluconsäure, Oxalsäure, Malonsäure, Acetessigsäure, Apfelsäure, Weinsäure, Ethylendiamintetraessigsäure (EDTA) und Mischungen davon.

In einer besonders bevorzugten Ausgestaltung der Erfindung kann vorgesehen sein, dass der zumindest eine Hilfsstoff eine Carbonsäure ist, ausgewählt aus der Gruppe bestehend aus Glyoxylsäure, Propansäure, Maleinsäure, 3-Butensäure, 2-Oxobutansäure, Methylmalonsäure, Itaconsäure, 2-Oxoglutarsäure, Dimethylmalonsäure, Glutarsäure, Methylbernsteinsäure, Adipinsäure, D-Glucarsäure, Hexansäure, Liponsäure, Furan-2-carbonsäure, Acrylsäure, Picolinsäure, Isocitronensäure, 4-Methylvaleriansäure, Asparaginsäure, Glutaminsäure, N-Acetylneuraminsäure, Niacin und Mischungen davon.

In einer weiter bevorzugten Ausgestaltung der Erfindung kann vorgesehen sein, dass der zumindest ein Hilfsstoff eine Carbonsäure ist, ausgewählt aus der Gruppe bestehend aus Glyoxylsäure, Propansäure, 2-Oxoglutarsäure, Acrylsäure und Mischungen davon.

In einer bevorzugten Ausgestaltung der Erfindung kann vorgesehen sein, dass der zumindest eine Hilfsstoff eine Sulfonsäure der folgenden Formel ist:

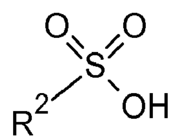

wobei $R^2$ C1-8-Alkyl, C2-8-Alkenyl, oder Cl-8-Aminoalkyl

ist.

Es konnte in überraschender Weise gezeigt werden, dass sich diese Stoffe besonders gut als Hilfsstoffe eignen.

In einer bevorzugten Ausgestaltung der Erfindung kann vorgesehen sein, dass der zumindest eine Hilfsstoff eine Sulfonsäure ist, ausgewählt aus der Gruppe bestehend aus Ethansulfonsäure, Methansulfonsäure, Taurin, und Mischungen davon.

In einer bevorzugten Ausgestaltung der Erfindung kann vorgesehen sein, dass der zumindest eine Hilfsstoff ein Enol ist, ausgewählt aus der Gruppe bestehend aus Barbitursäure, Ascorbinsäure, und Mischungen davon.

Dadurch kann vorteilhafter Weise erreicht werden, dass mit der Zeit keine unerwünschten Veresterungsreaktionen in der Verdampfungszusammensetzung vorkommen. Dadurch kann insbesondere die Lagerstabilität verbessert werden. Zudem kann dadurch auch die Verdampfungszusammensetzung vor unerwünschter Oxidation geschützt werden.

In einer bevorzugten Ausgestaltung der Erfindung kann vorgesehen sein, dass die Mischung zusätzlich zumindest einen Pufferbildner aufweist, wobei der Pufferbildner eine korrespondierende Base des zumindest einen Hilfsstoffes ist.

Dadurch kann vorteilhafter Weise erreicht werden, dass die Verdampfungszusammensetzung gepuffert ist. Dadurch kann der pH-Wert der Verdampfungszusammensetzung besonders gut auf einen gewünschten Wert eingestellt werden. Insbesondere kann der pH-Wert im Wesentlichen unabhängig von der Nikotinkonzentration eingestellt werden.

In einer bevorzugten Ausgestaltung der Erfindung kann vorgesehen sein, dass der Pufferbildner ein Salz ist, vorzugsweise ausgewählt aus der Gruppe bestehend aus Ammonium-, Lithium-, Natrium-, Kalium-, Magnesium- und/oder Calcium-Salzen.

In einer bevorzugten Ausgestaltung der Erfindung kann vorgesehen sein, dass die Verdampfungszusammensetzung ein molares Verhältnis von Pufferbildner zum zumindest einen Hilfsstoff aufweist in einem Bereich von größer oder gleich 0,25:1 bis kleiner oder gleich 2:1, vorzugsweise von größer oder gleich 0,5:1 bis kleiner oder gleich 1,5:1, besonders bevorzugt von größer oder gleich 0,9:1 zu kleiner oder gleich 1,1:1.

Dadurch kann vorteilhafter Weise erreicht werden, dass der Puffer besonders stabil ist und eine gute Pufferkapazität für das Nikotin aufweist.

In einer bevorzugten Ausgestaltung der Erfindung kann vorgesehen sein, dass die Zusammensetzung den zumindest einen Hilfsstoff und den zumindest einen Pufferbildner zusammen in einer Menge aufweist, die die Menge des Nikotins in der Zusammensetzung abpuffern kann.

Insbesondere ist darunter zu verstehen, dass die Pufferkapazität des Puffers so eingestellt ist, dass sich der pH-Wert durch die Zugabe des Nikotins um weniger als 1 ändert.

In einer bevorzugten Ausgestaltung der Erfindung kann vorgesehen sein, dass die Basisflüssigkeit eine Zusammensetzung aufweist bestehend aus Glycerin, Wasser, Propylenglykol, und/oder Mischungen.

Dadurch kann vorteilhafter Weise erreicht werden, dass die Verdampfungszusammensetzung besonders gut mit üblichen Mitteln, wie beispielsweise elektronischen Zigaretten, verdampft werden kann.

In einer bevorzugten Ausgestaltung der Erfindung kann vorgesehen sein, dass die Basisflüssigkeit eine Zusammensetzung aufweist bestehend aus Propylenglykol in einem Bereich von größer oder gleich 0 gew.-% bis kleiner oder gleich 90 gew.-%, Glycerin in einem Bereich von größer oder gleich 0 gew.-% bis kleiner oder gleich 90 gew.-% und Wasser in einem Bereich von größer oder gleich 0 gew.-% bis kleiner oder gleich 20 gew.-%.

Beispielsweise kann die Verdampfungszusammensetzung vorteilhafter Weise ein Massenverhältnis von Propylenglykol zu Glycerin zu Wasser aufweisen von 55:35:10, alternativ von 50:50:0, weiter alternativ von 25:75:0, oder von 30:70:0, 0:80:20, oder auch 90:0:10.

In einer bevorzugten Ausgestaltung der Erfindung kann vorgesehen sein, dass die Zusammensetzung ein für bekannte Verdampfungszusammensetzung übliches Aroma aufweist. Darunter sind auch Mischungen einer Mehrzahl von Aromen zu verstehen.

Dadurch kann vorteilhafter Weise erreicht werden, dass die Verdampfungszusammensetzung einen angenehmen Geschmack und/oder Geruch aufweist. Insbesondere kann durch die erfindungsgemäße Verdampfungszusammensetzung vorteilhafter weise erreicht werden, dass das Nikotin das Aroma nicht negativ beeinträchtigt.

Mit der Erfindung wird ferner die Verwendung einer vorbeschriebenen Verdampfungszusammensetzung zum Inhalieren beschrieben.

Im Detail ist vorgesehen, dass die Verdampfungszusammensetzung zum Inhalieren verwendet wird zur Einstellung der Resorption von Nikotin, zur Einstellung des Throat-Hits, und/oder zur Einstellung des Geschmacks.

Dadurch kann vorteilhafter Weise erreicht werden, dass beispielsweise eine Verdampfungszusammensetzung mit niedrigem Nikotingehalt trotzdem einen angenehmen Throat-Hit erzeugen kann oder alternativ eine Verdampfungszusammensetzung mit hohem Nikotingehalt trotzdem nur einen schwachen Throat-Hit erzeugt.

**Patentansprüche**

1. Verdampfungszusammensetzungen zum Inhalieren, bestehend zumindest aus

   - Nikotin,

- zumindest einen Hilfsstoff,
- optional Aroma,
- und als Rest eine Basisflüssigkeit,

wobei der zumindest eine Hilfsstoff zumindest einen ersten pKs-Wert aufweist in einem Bereich von größer oder gleich -5 bis kleiner oder gleich 10, vorzugsweise größer oder gleich -2 bis kleiner oder gleich 5, besonders bevorzugt größer oder gleich -2 bis kleiner oder gleich 4.

**2.** Verdampfungszusammensetzungen zum Inhalieren nach Anspruch 1, wobei der zumindest eine Hilfsstoff einen zweiten und optional dritten pKs-Wert aufweist in einem Bereich von größer oder gleich 0 bis kleiner oder gleich 10, vorzugsweise größer oder gleich 2 bis kleiner oder gleich 8, besonders bevorzugt größer oder gleich 3,5 bis kleiner oder gleich 6,5.

**3.** Verdampfungszusammensetzungen zum Inhalieren nach einem der vorherigen Ansprüche, wobei die Zusammensetzung das Nikotin in einer Menge in einem Bereich von größer oder gleich 1 mg/mL bis kleiner oder gleich 100 mg/mL bezogen auf die Zusammensetzung aufweist, vorzugsweise in einem Bereich von größer oder gleich 2 mg/mL bis kleiner oder gleich 20 mg/mL, beispielsweise in einem Bereich von größer oder gleich 4 mg/mL bis kleiner oder gleich 10 mg/mL.

**4.** Verdampfungszusammensetzungen zum Inhalieren nach einem der vorherigen Ansprüche, wobei die Zusammensetzung ein molares Verhältnis von Hilfsstoff zu Nikotin aufweist in einem Bereich von größer oder gleich 0,25:n bis kleiner oder gleich 4:n, vorzugsweise in einem Bereich von größer oder gleich 0,5:n bis kleiner oder gleich 2:n, besonders bevorzugt in einem Bereich von größer oder gleich 0,9:n bis kleiner oder gleich 1,1:n, wobei n die Anzahl der pKs-Werte des zumindest einen Hilfsstoffes nach einem der vorherigen Ansprüche ist.

**5.** Verdampfungszusammensetzungen zum Inhalieren nach einem der vorherigen Ansprüche, wobei der zumindest eine Hilfsstoff einen Siedepunkt und/oder Zersetzungspunkt in einem Bereich aufweist von größer oder gleich 80°C bis kleiner oder gleich 450°C, vorzugsweise größer oder gleich 90°C bis kleiner oder gleich 400°C, besonders bevorzugt von größer oder gleich 100°C bis kleiner oder gleich 300°C, insbesondere von größer oder gleich 110°C bis kleiner oder gleich 160°C.

**6.** Verdampfungszusammensetzungen zum Inhalieren nach einem der vorherigen Ansprüche, wobei der zumindest eine Hilfsstoff ausgewählt ist aus der Gruppe bestehend aus Carbonsäuren, Sulfonsäuren, Schwefelsäureestern, Phosphonsäuren, Phosphorsäureestern, CH-aciden Verbindungen, NH-aciden Verbindungen, Enolen, Phenolen, oder Mischungen davon.

**7.** Verdampfungszusammensetzungen zum Inhalieren nach einem der vorherigen Ansprüche, wobei der zumindest eine Hilfsstoff eine Carbonsäure der folgenden Formel ist:

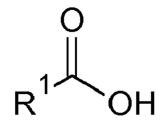

wobei $R^1$-H, -OH, -COOH, C1-8-Alkyl, C2-8-Alkenyl, C1-8-Ketoalkyl, C1-8-Aminoalkyl, Pyridyl, Furan, C1-8-Carboxyalkyl, C2-8-Carboxyalkenyl, C1-8-Ketocarboxyalkyl, C1-8-Aminocarboxalkyl, C1-8-Hydroxycarboxyalkyl, C1-8-Dicarboxyalkyl, C1-8-Hydroxydicarboxyalkyl, N-Neuraminsäure oder zyklisches Hydroxymethyltriterpenoidyl ist.

**8.** Verdampfungszusammensetzungen zum Inhalieren nach einem der vorherigen Ansprüche, wobei der zumindest eine Hilfsstoff eine Carbonsäure ist, ausgewählt aus der Gruppe bestehend aus Glyoxylsäure, Propansäure, Maleinsäure, 3-Butensäure, 2-Oxobutansäure, Methylmalonsäure, Itaconsäure, 2-Oxoglutarsäure, Dimethylmalonsäure, Glutarsäure, Methylbernsteinsäure, Adipinsäure, D-Glucarsäure, Hexansäure, Liponsäure, Furan-2-carbonsäure, Acrylsäure, Picolinsäure, Isocitronensäure, 4-Methylvaleriansäure, Asparaginsäure, Glutaminsäure, N-Acetylneuraminsäure, Niacin und Mischungen davon.

**9.** Verdampfungszusammensetzungen zum Inhalieren nach einem der vorherigen Ansprüche, wobei der zumindest eine Hilfsstoff eine Sulfonsäure der folgenden Formel ist:

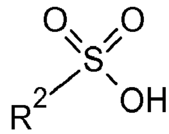

wobei $R^2$ C1-8-Alkyl, C2-8-Alkenyl, oder C1-8-Aminoalkyl ist.

**10.** Verdampfungszusammensetzungen zum Inhalieren nach einem der vorherigen Ansprüche, wobei der zumindest eine Hilfsstoff eine Sulfonsäure ist, ausgewählt aus der Gruppe bestehend aus Ethansulfonsäure, Methansulfonsäure, Taurin, und Mischungen davon.

**11.** Verdampfungszusammensetzungen zum Inhalieren nach einem der vorherigen Ansprüche, wobei der zumindest eine Hilfsstoff ein Enol ist, ausgewählt aus der Gruppe bestehend aus Barbitursäure, Ascorbinsäure, und Mischungen davon.

**12.** Verdampfungszusammensetzungen zum Inhalieren nach einem der vorherigen Ansprüche, wobei die Mischung zusätzlich zumindest einen Pufferbildner aufweist, wobei der Pufferbildner eine korrespondierende Base des zumindest einen Hilfsstoffes ist.

**13.** Verdampfungszusammensetzungen zum Inhalieren nach einem der vorherigen Ansprüche, wobei die Zusammensetzung den zumindest einen Hilfsstoff und den zumindest einen Pufferbildner zusammen in einer Menge aufweist, die die Menge des Nikotins in der Zusammensetzung abpuffern kann.

**14.** Verdampfungszusammensetzungen zum Inhalieren nach einem der vorherigen Ansprüche, wobei die Basisflüssigkeit eine Zusammensetzung aufweist bestehend aus Glycerin, Wasser, Propylenglykol, und/oder Mischungen.

**15.** Verwendung einer Verdampfungszusammensetzungen zum Inhalieren nach einem der vorherigen Ansprüche zur Einstellung der Resorption von Nikotin, zur Einstellung des Throat-Hits, und/oder zur Einstellung des Geschmacks.

Europäisches Patentamt
European Patent Office
Office européen des brevets

# EUROPÄISCHER RECHERCHENBERICHT

Nummer der Anmeldung
EP 19 16 6138

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (IPC) |
|---|---|---|---|
| X | WO 2019/005889 A1 (NUDE NICOTINE INC [US]; RUBENSTEIN JACOB [US]) 3. Januar 2019 (2019-01-03) * Ansprüche 1-39; Tabellen 1-13 * ----- | 1-15 | INV. A61K9/08 A24D3/14 A24F47/00 A61K47/12 A61K47/20 A61K47/24 |
| X | US 2016/044968 A1 (BOWEN ADAM [US] ET AL) 18. Februar 2016 (2016-02-18) * Absätze [0003], [0007], [0111], [0123], [0131], [0405], [0408]; Ansprüche 1-20; Beispiel 1; Tabelle 3 * ----- | 1-15 | |
| X | CN 108 030 143 A (WUHAN HUANGHELOU FLAVOR & SPICES CO LTD) 15. Mai 2018 (2018-05-15) * Beispiele 1-7 * ----- | 1-15 | |
| X | US 2017/334881 A1 (DULL GARY M [US] ET AL) 23. November 2017 (2017-11-23) * Anspruch 15 * ----- | 1-15 | |

| RECHERCHIERTE SACHGEBIETE (IPC) |
|---|
| A61K A24F A24B A24D |

1

Der vorliegende Recherchenbericht wurde für alle Patentansprüche erstellt

| Recherchenort | Abschlußdatum der Recherche | Prüfer |
|---|---|---|
| München | 27. September 2019 | Wörth, Christian |

KATEGORIE DER GENANNTEN DOKUMENTE

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder nach dem Anmeldedatum veröffentlicht worden ist
D : in der Anmeldung angeführtes Dokument
L : aus anderen Gründen angeführtes Dokument
& : Mitglied der gleichen Patentfamilie, übereinstimmendes Dokument

EPO FORM 1503 03.82 (P04C03)

## ANHANG ZUM EUROPÄISCHEN RECHERCHENBERICHT ÜBER DIE EUROPÄISCHE PATENTANMELDUNG NR.

EP 19 16 6138

In diesem Anhang sind die Mitglieder der Patentfamilien der im obengenannten europäischen Recherchenbericht angeführten Patentdokumente angegeben.
Die Angaben über die Familienmitglieder entsprechen dem Stand der Datei des Europäischen Patentamts am
Diese Angaben dienen nur zur Unterrichtung und erfolgen ohne Gewähr.

27-09-2019

| Im Recherchenbericht angeführtes Patentdokument | Datum der Veröffentlichung | Mitglied(er) der Patentfamilie | Datum der Veröffentlichung |
|---|---|---|---|
| WO 2019005889 A1 | 03-01-2019 | KEINE | |
| US 2016044968 A1 | 18-02-2016 | AU 2014262808 A1 | 12-11-2015 |
| | | AU 2019201728 A1 | 04-04-2019 |
| | | CA 2909967 A1 | 13-11-2014 |
| | | CN 105263345 A | 20-01-2016 |
| | | EP 2993999 A1 | 16-03-2016 |
| | | HK 1222516 A1 | 07-07-2017 |
| | | JP 6400678 B2 | 03-10-2018 |
| | | JP 2016520061 A | 11-07-2016 |
| | | JP 2019030301 A | 28-02-2019 |
| | | KR 20160004298 A | 12-01-2016 |
| | | RU 2015146869 A | 15-06-2017 |
| | | US 2014345631 A1 | 27-11-2014 |
| | | US 2015020824 A1 | 22-01-2015 |
| | | US 2016044967 A1 | 18-02-2016 |
| | | US 2016044968 A1 | 18-02-2016 |
| | | WO 2014182736 A1 | 13-11-2014 |
| CN 108030143 A | 15-05-2018 | KEINE | |
| US 2017334881 A1 | 23-11-2017 | CN 106536501 A | 22-03-2017 |
| | | EP 3148982 A1 | 05-04-2017 |
| | | JP 2017523133 A | 17-08-2017 |
| | | US 2015344456 A1 | 03-12-2015 |
| | | US 2017334881 A1 | 23-11-2017 |
| | | WO 2015183801 A1 | 03-12-2015 |

EPO FORM P0461

Für nähere Einzelheiten zu diesem Anhang : siehe Amtsblatt des Europäischen Patentamts, Nr.12/82